(19) Europäisches Patentamt
European Patent Office
Office européen des brevets

(11) **EP 4 218 711 A2**

(12) **EUROPEAN PATENT APPLICATION**

(43) Date of publication:
**02.08.2023   Bulletin 2023/31**

(21) Application number: **23159115.7**

(22) Date of filing: **02.09.2015**

(51) International Patent Classification (IPC):
*A61K 8/29* (2006.01)          *A61K 8/895* (2006.01)
*A61K 8/02* (2006.01)          *A61Q 17/04* (2006.01)

(52) Cooperative Patent Classification (CPC):
**A61K 8/29; A61K 8/0241; A61K 8/895; A61Q 17/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **02.09.2014   US 201462044490 P**

(62) Document number(s) of the earlier application(s) in accordance with Art. 76 EPC:
**15763175.5 / 3 188 708**

(71) Applicant: **Edgewell Personal Care Brands, LLC St. Louis MO 63017 (US)**

(72) Inventors:
• **LI, Geng**
  **Rutherford, New Jersey, 07070 (US)**
• **SANOGUEIRA, James**
  **Wesley Hills, New York, 10901 (US)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

Remarks:
This application was filed on 28-02-2023 as a divisional application to the application mentioned under INID code 62.

(54) **MINERAL SUNSCREEN COMPOSITIONS**

(57)    The disclosure relates to a mineral sunscreen composition having a synergistic combination of titanium dioxide and a film former that provides a Sun Protection Factor (SPF) of about 80 to about 200, and meets the FDA Critical Wavelength test method. The compositions have fluidity and self-spreading properties that leave a light, silky finish.

EP 4 218 711 A2

**Description**

CROSS REFERENCE TO RELATED APPLICATIONS

This application claims priority to the U.S. Provisional Application Serial Number 62/044490, filed on September 2, 2015, the content of which is incorporated herein for reference in its entirety.

BACKGROUND OF THE INVENTION

A. Field of Endeavor

The present invention relates to mineral sunscreen compositions with self-leveling and self-spreading properties, and to methods of using the composition to protect keratinous substrates such as skin and hair against ultraviolet radiation.

B. Background Information

Topical photoprotective agents, commonly known as sunscreens, must address two factors in order to provide effective sun protection. First, it should provide broad spectrum uniform protection against ultraviolet (UV) radiation in the UVA region (320 to 400 nm) and UVB region (290 to 320 nm). The UVA region is further divided between the shorter wavelengths of UVA-II radiation from 320 to 340 nm, and the slightly longer wavelengths of UVA-I radiation from 340 to 400 nm. Protection from UVA radiation is measured as a Protection Factor - UVA or PFA. Protection from UVB radiation is measured as a Sun Protection Factor or SPF.

The second factor for effective sun protection is user compliance. The sunscreen should have a pleasing sensory and tactile profile to enhance the user experience and promote compliance in use. For optimum sun protection, a user needs to re-apply the sunscreen every two hours. Sunscreen compositions that contain physical particles, namely mineral sunscreens, also referred to as sunblocks, containing titanium dioxide and/or zinc oxide are often thick, pasty, and have an unpleasant heavy feel during application. After application, they form an undesirable whiteness on the skin. These factors do result in diminished user compliance. While mineral sunscreens provide uniform protection against UV radiation well into the UVA-I region to fulfill the critical wavelength requirement of the FDA monograph more readily than organic sunscreens using a single sunscreen active, sun protection is insufficient if the sensory experience is a barrier to user compliance.

Thus, it is desirable to achieve a sunscreen composition using one or more mineral sunscreen actives that provides consumers with an improved sensory experience that provides enhanced sun protection against both UVA and UVB rays.

SUMMARY

In a first aspect, the present invention is directed to a sunscreen composition comprising an anhydrous combination comprising (i) titanium dioxide and (ii) one or more film forming polymers, wherein the sunscreen composition has a spreading value of at least about 10 cm$^2$ at 20 seconds without rub out. The sunscreen composition may also have a spreading value of at least about 18 cm$^2$ at 20 seconds without rub out. Preferably, the sunscreen composition is substantially emulsion-free. Preferably, the titanium dioxide may be present in an amount of up to about 40.0 wt. %; more preferably, the titanium dioxide may be present in an amount of up to about 30.0 wt. %; and most preferably, the titanium dioxide may be present in an amount of up to about 20.0 wt.%. Preferably, the one or more film formers are selected from the group consisting of acrylate copolymers, polyester copolymers, polyolefin copolymers, silicone copolymers, and combinations thereof. More preferably, the one or more film formers may comprise acrylate copolymers.

Preferably, the sunscreen composition may further include a diluent. The diluent may be selected from the group consisting of isododecane, dodecane, tridecane, isohexadecane, pentadecane, and combinations thereof. More preferably, the diluent comprises isododecane.

Preferably, the sunscreen composition may further include an additional sunscreen agent. The additional sunscreen agent may comprise zinc oxide. The additional sunscreen agent may also comprise one or more organic sunscreen agents selected from the group consisting of p-aminobenzoic acid and derivatives thereof; butyl methoxydibenzoylmethane; benzophenones; hydroxy-substituted benzophenones; methoxy-substituted benzophenones; benzophonone-1; benzophenone-2; benzophenone-3; benzophenone-4; benzophenone-6; benzophenone-8; benzophenone-12; methoxycinnamate; ethyl dihydroxypropyl-p-aminobenzoate; glyceryl-p-aminobenzoate; homosalate; methyl anthranilate; octocrylene; octyl dimethyl-p-aminobenzoate; octyl methoxycinnamate; octyl salicylate; 2 phenylbenzimidazole-5-sulphonic acid; triethanolamine salicylate; 3-(4-methylbenzylidene)-camphor; red petrolatum,3-(4-methylbenzyldine)boran-2-one(methylbenzindinecamphor); benzotriazole; salicylates; phenylbenzimidazole-5-sulfonic acid; methylene bis-benzotriazolyl tetramethylbutyl phenol; avobenzone; 4-isopropyldibenzoylmethane; butylmethoxydibenzoylmethane; octisalate; oxybenzone; bis-ethylhexyloxyphenol methoxy triazine; 4-isopropyl-dibenzoylmethane; metal oxides; zinc oxide; octyltriethoxy silanol; alumina; triethoxy silane; and combinations thereof. Preferably, the additional sunscreen agent may comprise one or more organic sunscreen agents selected from the group consisting of octocrylene, homosalate, octisalate, and combinations thereof.

In another aspect, the present invention is directed to a sunscreen composition consisting essentially of a synergistic combination comprising (i) titanium dioxide is present in an amount of up to about 40.0 wt. %; (ii) a film former comprising acrylate copolymers, polyester copolymers, polyolefin copolymers, silicone copolymers, or combinations thereof; and (iii) a diluent, wherein the sunscreen composition has a spreading value of at least about 10 $cm^2$ at 20 seconds without rub out. Preferably, the sunscreen composition has a spreading value of at least about 20 $cm^2$ at 20 seconds without rub out. Preferably, the film former may comprise cyclopentasiloxane (and) acrylate/dimethicone copolymers. More preferably, the film former is present in an amount of about 0.05 wt. % to about 5.0 wt. %. Most preferably, the film former is present in an amount of about 1.0 wt. % to about 2.0 wt. %. Preferably, the titanium dioxide is present in an amount of up to about 20.0 wt. %. More preferably, the sunscreen composition may further include zinc oxide. Preferably, the diluent is selected from the group consisting of isododecane, dodecane, tridecane, isohexadecane, pentadecane, and combinations thereof.

In yet another aspect, the present invention is directed to a sunscreen composition consisting essentially of (i) a synergistic combination comprising a therapeutic amount of titanium dioxide to provide an SPF of about 80 to about 200, and a film former comprising acrylate copolymers, polyester copolymers, polyolefin copolymers, silicone copolymers, or combinations thereof; (ii) a diluent; and (iii) an additional sunscreen agent, wherein the sunscreen composition has a spreading value of at least about 20 $cm^2$ at 20 seconds without rub out.

In still yet another aspect, the present invention is directed to a consumer packaged product comprising a sunscreen composition of any of the embodiments disclosed herein.

In still yet another aspect, the present invention is directed to a method of protecting keratinous substrates from ultraviolet radiation by applying a therapeutic amount of any sunscreen composition disclosed herein to the keratinous substrate.

In still yet another aspect, the present invention is directed to a method of absorbing ultraviolet radiation by applying any sunscreen composition disclosed herein to a keratinous substrate and subjecting the keratinous substrate to ultraviolet radiation.

## DETAILED DESCRIPTION OF THE INVENTION

The embodiments of the present invention can comprise, consist of, and consist essentially of the features and/or steps of the invention described herein, as well as any of the additional or optional ingredients, components, steps, or limitations described herein or would otherwise be appreciated by one of skill in the art. It is to be understood that all concentrations disclosed herein are by weight percent (wt. %) based on a total weight of the composition unless otherwise indicated. Where appropriate, the INCI (International Nomenclature of Cosmetic Ingredients) name of ingredients/components is used.

The present invention is directed to a sunscreen composition containing a mineral sunscreen agent, which has improved spreadability due to its self-leveling properties, provides a light and silky feel on a keratinous substrate, and unexpectedly fulfills the FDA Critical Wavelength test method to achieve a critical wavelength of ≥370 nm for broad spectrum protection. The sunscreen composition includes the following: an anhydrous combination comprising a therapeutic amount of a mineral sunscreen agent such as titanium dioxide and a film former. Preferably, the composition also includes a diluent. It may further comprise a variety of additional components to provide additional functionality to the user. The unexpected synergy of the mineral sunscreen agent, preferably titanium dioxide, and the film former provides broad spectrum protection against UVB through UVA-I radiation up to and beyond the critical wavelength of 370 nm when used alone or in combination with additional sunscreen agents. The sunscreen compositions of the present invention can be formulated with pharmaceutically acceptable carrier components to provide different formulations as a lotion or spray product. A further embodiment provides a consumer packaged product wherein a porous applicator can be used to apply the sunscreen composition on a keratinous substrate surface by a user.

Titanium dioxide useful in the aforementioned sunscreen compositions is preferably available as an anhydrous slurry or paste in a hydrocarbon carrier. The titanium dioxide particles themselves typically have an inert coating to reduce its photocatalytic activity. Some titanium dioxide particles have one or more coatings. Preferably, the titanium dioxide particles useful in the disclosed formulation have at least one coating comprising silicone copolymers; fatty acids or salts thereof; oxides of silicon, aluminum, zirconium, and the like; and combinations thereof. By choosing a coating that is compatible with the carrier, the resulting formulation enjoys enhanced stability and the titanium dioxide particles remain well dispersed in the sunscreen composition with no particle settling.

Preferred embodiments utilize titanium dioxide sold under the trade name SiClone® TD-150 by Presperse Corporation, Somerset, New Jersey. The SiClone TD-150 product contains about 40% titanium dioxide (solids content) with an inner coating of aluminum hydroxide and an outer coating of isostearic acid in a hydrocarbon carrier (isohexadecane/isododecane/$C_{13}$ to $C_{15}$ alkanes) that is substantially free of any wetting agents. The titanium dioxide is preferably present in a therapeutically effective amount to provide an SPF of greater than 77 and a critical wavelength of at least 371 nm; more preferably in an amount of about 15.0 wt. % to about 40.0 wt. %, and more preferably in an amount of about 15.0 wt. % to about 20.0 wt. %, all based on a total weight of the sunscreen composition.

The film formers useful in the anhydrous combination of the sunscreen composition disclosed herein provide a boosting effect in the SPF of the titanium dioxide. The SPF of the titanium dioxide, neat SiClone TD-150, is about 77. By adding a preferred film former, the SPF is boosted by about 6% to about 80% or more exhibiting a synergistic effect between the preferred film former and the titanium dioxide since the film former itself does not register an SPF. When an additional sunscreen agent is added into the formulation, the SPF can be further increased by about 250% or more depending on the particular additional sunscreen agent and amounts thereof. In a most preferred embodiment, the SPF is boosted by the addition of the film former by at least about 6%, and in some embodiments by at least about 80%.

Preferred film formers can be selected from the group consisting of acrylate copolymers, polyester copolymers, polyolefin copolymers, and combinations thereof. Exemplary acrylate copolymers are silicone acrylates such as, but not limited to, cyclopentasiloxane (and) acrylate/dimethicone copolymer; acrylic acid/isobornyl methacrylate/isobutyl methacrylate copolymer/isododecane; isododecane (and) acrylate/dimethicone copolymer; and combinations thereof. Exemplary of a polyester copolymer useful in the present disclosure is octyldodecyl citrate crosspolymer. Exemplary of polyolefin polymers useful in the present disclosure are isododecane (and) butylene/ethylene/propylene copolymer; and hydrogenated polycyclopentadiene (and) isododecane. The above lists are only examples and non-limiting.

Most preferred film formers comprise acrylate copolymers. Commercially available silicone acrylate copolymers are sold under the trade name KP-545 from Shinetsu Corporation, Japan; GIOVAREZ® AC-34M from Phoenix Chemical, Inc. of Somerville, New Jersey; and CHEMSIL® K-50 from ChemSil Silicones, Inc. of Chatsworth, California. The film former can be present in an amount sufficient to boost the SPF of the titanium dioxide by at least 6% and, in some cases, over 80%, and up to 250% is also possible. Preferably, the film former is present in an amount of about 0.05 wt. % to about 5.0 wt. %, based on a total weight of the sunscreen composition. More preferably, the film former is present in an amount of about 1.0 wt. % to about 3.0 wt. %, based on a total weight of the sunscreen composition. Most preferably, the film former is present in an amount of up to about 1.0 wt. % to about 2.0 wt. %, based on a total weight of the sunscreen composition.

The sunscreen composition may further include a chemically compatible non-aqueous diluent. Preferably the diluent is a hydrocarbon diluent. It can be selected from the group consisting of isododecane, dodecane, tridecane, isohexadecane, pentadecane, and combinations thereof. Preferred diluents are isododecane; isohexadecane/isododecane/$C_{13}$ to $C_{15}$ alkane; pentadecane; dodecane/tridecane. The above lists are only examples and are not limiting. Commercially available diluents are sold under the trade name PERMETHYL® 99A and SiClone SR5, both from Presperse Corporation; and ELEVANCE® Soft from Elevance Renewable Sciences, Inc.

The diluent is preferably present in a quantity sufficient (q.s.) to bring the total amount of titanium dioxide and film former to 100.0 g. Depending on the final composition, the diluent may be present in an amount of up to about 95.0 wt. %, more preferably based on an amount of about 25.0 wt. % to about 95.0 wt. %, and most preferably based on an amount of about 25.0 wt. % to about 50.0 wt. %, all based on a total weight of the sunscreen composition.

The sunscreen composition of the present disclosure may further include additional sunscreen agents such as zinc oxide or organic sunscreen agents, alone or in combination. Organic sunscreen agents that can be used in the sunscreen composition include, but are not limited to p-aminobenzoic acid and derivatives thereof; butyl methoxydibenzoylmethane; benzophenones; hydroxy-substituted benzophenones; methoxy-substituted benzophenones; benzophenone-1; benzophenone-2; benzophenone-3; benzophenone-4; benzophenone-6; benzophenone-8; benzophenone-12; methoxycinnamate; ethyl dihydroxypropyl-p-aminobenzoate; glyceryl-p-aminobenzoate; homosalate; methyl anthranilate; octocrylene; octyl dimethyl-p-aminobenzoate; octyl methoxycinnamate; octyl salicylate; 2 phenylbenzimidazole-5-sulphonic acid; triethanolamine salicylate; 3-(4-methylbenzylidene)-camphor; red petrolatum,3-(4-methylbenzyldine)boran-2-one(methylbenzindinecamphor); benzotriazole; salicylates; phenylbenzimidazole-5-sulfonic acid; methylene bisbenzotriazolyl tetramethylbutyl phenol; avobenzone; 4-isopropyldibenzoylmethane; butylmethoxydibenzoylmethane; octisalate; oxybenzone; bis-ethylhexyloxyphenol methoxy triazine; 4-isopropyl-dibenzoylmethane; metal oxides; zinc oxide; octyltriethoxy silanol; alumina; triethoxy silane; and combinations thereof.

The preferred additional sunscreen agents are zinc oxide, avobenzone, octyl salicylate, homosalate, octocrylene, or combinations thereof. The one or more sunscreen agents can be included in a present composition at about 5.0 wt. % to about 35.0 wt. % based on a total weight of the composition. The amount and types of sunscreen agents in the composition will vary in the above range depending on the target SPF. The higher the SPF, the greater the total amount of sunscreen agents. Preferably, the one or more additional sunscreen agents are present in an amount of about 3.0 wt. % to about 30.0 wt. % to achieve a SPF of about 80 to about 200 and more, based on a total weight of the sunscreen composition. More preferably, the one or more additional sunscreen agents are present in an amount of about 3.0 wt. % to about 25.0 wt. %, based on a total weight of the sunscreen composition.

The cosmetic compositions of the present invention may optionally include other active or inactive ingredients such as those selected from, but not limited to, cosmetically acceptable carriers, oils, sterols, amino acids, moisturizers, powders, colorants (including pigments and/or dyes), pH adjusters, perfumes, essential oils, cosmetic active ingredients, vitamins, essential fatty acids, sphingolipids, self-tanning compounds such as dihydroxyacetone (DHA) and erythruloses, fillers, emulsifying agents, antioxidants, surfactants, additional film formers, chelating agents, gelling agents, thickeners, emollients, humectants, moisturizers, minerals, viscosity and/or rheology modifiers, keratolytics, retinoids, hormonal compounds, alpha-hydroxy acids, alpha-keto acids, anti-mycobacterial agents, anti-fungal agents, antimicrobials, anti-virals, analgesics, anti-allergenic agents, H1 or H2 antihistamines, antiinflammatory agents, anti-irritants, anti-neoplastics, immune system boosting agents, immune system suppressing agents, anti-acne agents, anesthetics, antiseptics, insect repellents, skin cooling compounds, skin protectants, skin penetration enhancers, exfoliants, lubricants, fragrances, colorants, staining agents, depigmenting agents, hypopigmenting agents, preservatives, stabilizers, pharmaceutical agents, photostabilizing agents, spherical powders, one or more fragrances, plant extracts, absorbents, salicylic acid, alpha and beta hydroxy acids, vitamins including vitamins A, C, and E, retinol and its derivatives, or any mixtures thereof. The above is a list of examples and is not meant to be limiting.

In some embodiments, the sunscreen agent is titanium dioxide wherein the particles have an inner coating of aluminum hydroxide and an outer coating of isostearic acid, the film former is cyclopentasiloxane (and) acrylate/dimethicone copolymer, and the diluent is isododecane.

In some embodiments, the sunscreen agent is titanium dioxide wherein the particles have an inner coating of aluminum hydroxide and an outer coating of isostearic acid, the film former is acrylic acid/isobornyl methacrylate/isobutyl methacrylate copolymer/isododecane, and the diluent is isododecane.

In some embodiments, the sunscreen agent is titanium dioxide wherein the particles have an inner coating of aluminum hydroxide and an outer coating of isostearic acid, the film former is cyclopentasiloxane (and) acrylate/dimethicone copolymer, and the diluent is isohexane/isododecane/$C_{13}$ to $C_{15}$ alkane.

In some embodiments, the sunscreen agent is titanium dioxide wherein the particles have an inner coating of aluminum hydroxide and an outer coating of isostearic acid, the film former is cyclopentasiloxane (and) acrylate/dimethicone copolymer, and the diluent is pentadecane.

In some embodiments, the sunscreen agent is titanium dioxide wherein the particles have an inner coating of aluminum hydroxide and an outer coating of isostearic acid, the film former is cyclopentasiloxane (and) acrylate/dimethicone copolymer, and the diluent is dodecane and tridecane.

In some embodiments, the sunscreen agents are zinc oxide and titanium dioxide wherein the titanium dioxide particles have an inner coating of aluminum hydroxide and an outer coating of isostearic acid, the film former is cyclopentasiloxane (and) acrylate/dimethicone copolymer, and the diluent is isododecane.

In some embodiments, the sunscreen agent is titanium dioxide wherein the particles have an inner coating of aluminum hydroxide and an outer coating of isostearic acid, the film former is isododecane (and) acrylate/dimethicone copolymer, and the diluent is isododecane.

In some embodiments, the sunscreen agent is titanium dioxide wherein the particles have an inner coating of aluminum hydroxide and an outer coating of isostearic acid, and the film former is cyclopentasiloxane (and) acrylate/dimethicone copolymer, wherein the composition is free of a separate diluent.

In some embodiments, the sunscreen agent is titanium dioxide wherein the particles have an inner coating of aluminum hydroxide and an outer coating of isostearic acid, the film former is cyclopentasiloxane (and) acrylate/dimethicone copolymer, the diluent is isododecane, and the additional sunscreen agents are homosalate, octisalate, octocrylene, and avobenzone.

The sunscreen compositions according to the present disclosure may be prepared according to techniques that are well known to one of ordinary skill in the art. The present disclosure will be better understood from the examples that follow, all of which are intended for illustrative purposes only and are not meant to limit the scope of the instant disclosure in any way.

Samples of the sunscreen composition of the present disclosure shown in Table I were prepared using the following method: to a clean and sanitized beaker were placed an amount of titanium dioxide slurry (SiClone TD-150 as a dispersion with about 40% solids content) and an amount of a film former. The titanium dioxide and film former were mixed together at about 70°C for about 20 minutes. The mixture was homogenized with stirring at 2500 rpm for about 10 minutes then cooled to about 40°C to which an amount of diluent was added. Again the mixture was homogenized with stirring at 2500 rpm until the mixture reached room temperature. In samples containing organic sunscreens agents, the organic sunscreens agents were mixed together in a separate vessel and heated to about 75°C until a clear mixture (neat) was obtained. The clear mixture of organic sunscreen agents was then added to the heated titanium dioxide mixture prior to homogenization and cooling to room temperature.

Preferably, the sunscreen composition of the present invention comprises a single anhydrous phase such that emulsifiers are not needed in formulating the inventive composition, nor is a multi-phase process required in a method of making the inventive composition.

Samples were prepared using 50.0 wt. % of the SiClone TD-50, which provided a solids content of about 20.0 wt. % to about 25 wt. % of titanium dioxide particles unless otherwise indicated in Table I; 0.05 wt. % to 5.0 wt. % film former was used unless otherwise indicated, and the specified diluent was used in a quantity sufficient to bring the weight of the composition to 100.0 g. Samples 8 to 10 have additional sunscreen agents. Sample 12 is a comparative example of a mineral sunscreen agent comprising only 5.0 wt. % zinc oxide (ZnO).

| TABLE I | | | |
|---|---|---|---|
| Sample No. | Film Former* | Diluent | Notes |
| 1 | A | isododecane | |
| 2 | B | isododecane | |
| 3 | B | isohexane/isododecane/ $C_{13}$-$C_{15}$ alkane | |
| 4 | B | pentadecane | |
| 5 | B | dodecane/tridecane | |
| 6 | C | isododecane | |
| 7 | B | - - | approx. 40% $TiO_2$ in dispersion |
| 8 | A | isododecane | +3 wt. % to 25 wt. % organic sunscreen mix of homosalate, octisalate, octocrylene, avobenzone |
| 9 | A | isododecane | 2 wt. % film former |
| 10 | A | isododecane | 3 wt. % film former |
| 11 | A | isododecane | + 5 wt. % ZnO |
| 12 | A | isododecane | No $TiO_2$; only sunscreen agent is 5 wt. % ZnO |
| *Film former A: GIOVAREZ AC-34M; Film former B: KP-545; Film Former C: CHEMSIL K-50 | | | |

While additional additives may be added, they are not required. As such, the sunscreen compositions of the present disclosure are incredibly simple to manufacture with fewer ingredients that may be sensitizing to consumers.

*In vitro* SPF and critical wavelength ($\lambda_c$) data were obtained using a Labsphere Ultraviolet Transmittance Analyzer (Model UV-2000 available from the Solar Light Company, Philadelphia, Pennsylvania) and presented in TABLE II. Samples of the sunscreen compositions weighing 1.6 mg/cm² were transferred by an adjustable pipette and uniformly applied to a Schonberg sand-blast PMMA plate (roughness 6 μm) by finger with a presaturated finger cot. After application, the coated plate was air dried for 15 minutes. The sample plate was then placed inside the Labsphere Analyzer. Irradiation took place at 4 randomly selected points. The readings were recorded by the analyzer and the calculation of the SPF value was based on the following equation:

$$SPF = \frac{\int\limits_{280nm}^{400nm} E_\lambda \cdot S_\lambda \cdot d\lambda}{\int\limits_{280nm}^{400nm} E_\lambda \cdot S_\lambda \cdot T_\lambda \cdot d\lambda}$$

with the use of built-in software: UV-2000 application Version 1.1.0.0, wherein $E_\lambda$ is defined as the Commission Internationale de l'Eclairage (CIE) erythemal spectral effectiveness, $S_\lambda$ is designated as the solar spectral irradiance, and $T_\lambda$ is the spectral transmittance of the sample as measured on the UV-2000. An average of four readings was recorded as the in-vitro SPF value of each sample shown in Table II.

| TABLE II | | |
|---|---|---|
| Sample No. | SPF | $\lambda_c$ |
| 1 | 89 | 372 |
| 2 | 91 | 371 |
| 3 | 93 | 367 |
| 4 | 91 | 367 |
| 5 | 93 | 367 |
| 6 | 82 | 371 |
| 7 | 139 | 370 |
| 8 | 193 | 378 |
| 9 | 95 | 372 |
| 10 | 92 | 373 |
| 11 | 104 | 370 |
| 12 | 3 | 377 |

The sunscreen compositions of Samples 1 to 11 show an increase in SPF when compared to the SPF of the neat SiClone TD-150 without any film former suggesting there is a boosting effect when the titanium dioxide is formulated with a selected film former in accordance with the present disclosure. In Samples 1 and 2, the SPF was boosted about 18% while also meeting the critical wavelength requirement. Sample 11 incorporates 5.0 wt. % of an additional sunscreen agent into the formulation, namely zinc oxide, to provide an even higher SPF than using zinc oxide alone as in Sample 12 that included the film former and diluent.

Spreading values of each of the above samples were determined by applying a 0.5 g sample to the back of a subject's hand without rubbing, and measuring the spread area after 20 seconds. Results are presented in TABLE III with comparative examples (Samples A, B, C, and D) also shown.

| TABLE III | |
|---|---|
| Sample No. | Spreading Value ($cm^2$) |
| 1 | 20 |
| 2 | 20 |
| 3 | 18 |
| 4 | 20 |
| 5 | 24 |
| 6 | 20 |
| 7 | 10 |
| 8 | 18 |
| 9 | 20 |
| 10 | 20 |
| 11 | 18 |
| 12 | 20 |
| A | 2 |
| B | 2 |
| C | 10 |

7

(continued)

| TABLE III | |
|---|---|
| Sample No. | Spreading Value (cm$^2$) |
| D | 10 |
| Sample A: Commercial Product containing 20.0 wt. % ZnO only<br>Sample B: Commercial Product containing 5.0 wt. % TiO$_2$ and 3.0 wt. % ZnO<br>Sample C: SiClone TD-150 neat containing 40.0% solids<br>Sample D: Commercial Product containing 42.0 wt. % TiO$_2$ only | |

Inventive Samples 1 to 6 and 8 to 12 demonstrate the exceptional self-spreading properties of the mineral sunscreen composition of the permanent invention when the sunscreen composition is formulated with the film formers disclosed herein. By incorporating one or more of the selected film formers into the mineral sunscreen formulation, the spreading values show about a ten-fold increase over the commercially available mineral sunscreen products, Samples A and B, making the inventive sunscreen composition easier to spread on the skin without tugging and leaving a light, silky finish.

The sunscreen compositions of the present invention can be used in a consumer packaged product as a lotion or formulated as a spray product. The viscosity of the sunscreen compositions is less than about 1000 cPs, and preferably less than 750 cPs. Given the low viscosity of the sunscreen composition, a spray product can be a finger pump product or a continuous spray product. The consumer packaged product may also comprise a porous plastic applicator. Such a product may contain a reservoir for storing the composition and a wick to draw the sunscreen composition from the reservoir to the applicator for easy application by a user.

A method of protecting a keratinous substrate such as the skin or hair would comprise applying a therapeutic amount of a sunscreen composition of the present invention to the substrate by a user. A method of absorbing ultraviolet radiation would comprise applying an amount of a sunscreen composition of the present invention by a user to a keratinous substrate and subjecting the keratinous substrate to ultraviolet radiation.

Sunscreen compositions containing mineral sunscreen agents, titanium dioxide and zinc oxide, are known to be thick and pasty. They are difficult to spread leaving a heavy, undesirable skin feel with a white chalky appearance after application. However, by utilizing one or more of the specific film formers disclosed herein, sunscreen compositions comprising titanium dioxide can have a light, silky finish and a synergistic effect is shown wherein the SPF, and preferably also the PFA, of the titanium dioxide is increased. The addition of an additional sunscreen agent such as zinc oxide and other organic sunscreen agents can provide further broad spectrum protection to keratinous substrates against damaging UV radiation.

While the present invention has been particularly described, in conjunction with specific preferred embodiments, it is evident that many alternatives, modifications and variations will be apparent to those skilled in the art in light of the foregoing description. It is therefore contemplated that the appended claims will embrace any such alternatives, modifications and variations as falling within the true scope and spirit of the present invention.

Items

Item 1.A sunscreen composition comprising
an anhydrous combination comprising

titanium dioxide and
one or more film forming polymers,

wherein said sunscreen composition has a spreading value of at least about 10 cm$^2$ at 20 seconds without rub out.

Item 2.The sunscreen composition of item 1 wherein said sunscreen composition is substantially emulsion-free.

Item 3. The sunscreen composition of item 1 wherein said sunscreen has a spreading value of at least about 18 cm$^2$ at 20 seconds without rub out.

Item 4.The sunscreen composition of item 1 wherein said titanium dioxide is present in an amount of up to about 40.0 wt. %.

Item 5. The sunscreen composition of item 1 wherein said titanium dioxide is present in an amount of up to about 30.0 wt. %.

Item 6. The sunscreen composition of item 1 wherein said titanium dioxide is present in an amount of up to about 20.0 wt. %.

Item 7. The sunscreen composition of item 1 wherein said one or more film formers are selected from the group consisting of acrylate copolymers, polyester copolymers, polyolefin copolymers, silicone copolymers, and combinations thereof.

Item 8. The sunscreen composition of item 7 wherein said one or more film formers comprises acrylate copolymers.

Item 9. The sunscreen composition of item 1 further including a diluent.

Item 10. The sunscreen composition of item 9 wherein said diluent is selected from the group consisting of isododecane, dodecane, tridecane, isohexadecane, pentadecane, and combinations thereof.

Item 11. The sunscreen composition of item 9 wherein said diluent comprises isododecane.

Item 12. The sunscreen composition of item 1 further including an additional sunscreen agent.

Item 13. The sunscreen composition of item 12 wherein said additional sunscreen agent comprises zinc oxide.

Item 14. The sunscreen composition of item 12 wherein said additional sunscreen agent comprises one or more organic sunscreen agents selected from the group consisting of p-aminobenzoic acid and derivatives thereof; butyl methoxydibenzoylmethane; benzophenones; hydroxy-substituted benzophenones; methoxy-substituted benzophenones; benzophonone-1; benzophenone-2; benzophenone-3; benzophenone-4; benzophenone-6; benzophenone-8; benzophenone-12; methoxycinnamate; ethyl dihydroxypropyl-p-aminobenzoate; glyceryl-p-aminobenzoate; homosalate; methyl anthranilate; octocrylene; octyl dimethyl-p-aminobenzoate; octyl methoxycinnamate; octyl salicylate; 2 phenylbenzimidazole-5-sulphonic acid; triethanolamine salicylate; 3-(4-methylbenzylidene)-camphor; red petrolatum,3-(4-methylbenzyldine)boran-2-one(methylbenzindinecamphor); benzotriazole; salicylates; phenylbenzimidazole-5-sulfonic acid; methylene bis-benzotriazolyl tetramethylbutyl phenol; avobenzone; 4-isopropyldibenzoylmethane; butylmethoxydibenzoylmethane; octisalate; oxybenzone; bis-ethylhexyloxyphenol methoxy triazine; 4-isopropyl-dibenzoylmethane; metal oxides; zinc oxide; octyltriethoxy silanol; alumina; triethoxy silane; and combinations thereof.

Item 15. The sunscreen composition of item 12 wherein said additional sunscreen agent comprises one or more organic sunscreen agents selected from the group consisting of octocrylene, homosalate, octisalate, and combinations thereof.

Item 16. The sunscreen composition of item 1 further including an apparatus for applying said sunscreen composition having a porous applicator portion and a wick.

Item 17. A consumer packaged product comprising a sunscreen composition of item 1.

Item 18. A method of protecting a keratinous substrate from ultraviolet radiation comprising applying a therapeutic amount of a sunscreen composition according to item 1 to said substrate.

Item 19. A method of absorbing ultraviolet radiation comprising applying a sunscreen composition according to item 1 to a keratinous substrate and subjecting the keratinous substrate to ultraviolet radiation.

Item 20. A sunscreen composition comprising

a synergistic combination consisting essentially of

titanium dioxide is present in an amount of up to about 40.0 wt. %; and

a film former comprising acrylate copolymers, polyester copolymers, polyolefin copolymers, silicone copolymers, or combinations thereof; and

a diluent,

wherein said sunscreen composition has a spreading value of at least about 10 cm$^2$ at 20 seconds without rub out.

Item 21. The sunscreen composition of item 20 wherein said composition has a spreading value of at least about 20 cm$^2$ at 20 seconds without rub out.

Item 22. The sunscreen composition of item 20 wherein said film former comprises cyclopentasiloxane (and) acrylate/dimethicone copolymers.

Item 23. The sunscreen composition of item 20 wherein said film former is present in an amount of about 0.05 wt. % to about 5.0 wt. %.

Item 24. The sunscreen composition of item 23 wherein said film former is present in an amount of about 1.0 wt. % to about 2.0 wt. %.

Item 25. The sunscreen composition of item 20 wherein said titanium dioxide is present in an amount of up to about 25.0 wt. %.

Item 26. The sunscreen composition of item 20 wherein said diluent is selected from the group consisting of isododecane, dodecane, tridecane, isohexadecane, pentadecane, and combinations thereof.

Item 27. The sunscreen composition of item 20 further including an additional sunscreen agent.

Item 28. The sunscreen composition of item 20 further including an apparatus for applying said sunscreen composition having a porous applicator portion and a wick.

Item 29. A consumer packaged product comprising a sunscreen composition of item 20.

Item 30. A method of protecting a keratinous substrate from ultraviolet radiation comprising applying a therapeutic amount of a sunscreen composition according to item 20 to said substrate.

Item 31. A method of absorbing ultraviolet radiation comprising applying a sunscreen composition according to item 20 to a keratinous substrate and subjecting the keratinous substrate to ultraviolet radiation.

Item 32. A sunscreen composition consisting essentially of

a synergistic combination comprising

a therapeutic amount of titanium dioxide to provide an SPF of about 80 to about 200, and
a film former comprising acrylate copolymers, polyester copolymers, polyolefin copolymers, silicone copolymers, or combinations thereof;

a diluent; and
an additional sunscreen agent,

wherein said sunscreen composition has a spreading value of at least about 20 cm$^2$ at 20 seconds without rub out.

Item 33. A consumer packaged product comprising a sunscreen composition of item 32.

Item 34. A method of protecting a keratinous substrate from ultraviolet radiation comprising applying a therapeutic amount of a sunscreen composition according to item 32 to said substrate.

Item 35. A method of absorbing ultraviolet radiation comprising applying a sunscreen composition according to item 32 to a keratinous substrate and subjecting the keratinous substrate to ultraviolet radiation.

**Claims**

1.  A sunscreen composition comprising:
    an anhydrous combination comprising:

    a mineral sunscreen agent;
    a film former selected from polyester copolymers; and

    a non-aqueous diluent, wherein the non-aqueous diluent is present in an amount up to and including 95 wt. %, based on a total weight of the sunscreen composition.

2.  The sunscreen composition of claim 1, wherein the mineral sunscreen agent includes titanium dioxide, zinc oxide, or combinations thereof.

3.  The sunscreen composition of claim 2, wherein the titanium dioxide has at least one coating comprising silicone copolymers, fatty acids, salts of fatty acids, oxides of silicon, oxides of aluminum, oxides of zirconium, or combinations thereof.

4.  The sunscreen composition of claim 1, wherein the titanium dioxide is present in an amount up to and including 20 wt. %, based on a total weight of the sunscreen composition.

5.  The sunscreen composition of claim 1, wherein the zinc oxide is present in an amount of 5 wt. % to 30 wt. %, based on a total weight of the sunscreen composition.

6.  The sunscreen composition of claim 1, wherein the polyester copolymers include octyldodecyl citrate crosspolymer.

7.  The sunscreen composition of claim 1, wherein the film former is further selected from acrylate copolymers.

8.  The sunscreen composition of claim 1, wherein the polyester copolymers are present in an amount of 0.05 wt. % to 5 wt. %, based on a total weight of the sunscreen composition.

9.  The sunscreen composition of claim 1, wherein the non-aqueous diluent is selected from a hydrocarbon comprising isododecane, dodecane, tridecane, isohexadecane, pentadecane, or combinations thereof.

10. The sunscreen composition of claim 9, wherein the hydrocarbon comprises isododecane.

11. The sunscreen composition of claim 1, wherein the non-aqueous diluent is present in an amount of 25 wt.% to 50 wt. %, based on a total weight of the sunscreen composition.

12. The sunscreen composition of claim 1, further including an organic sunscreen agent.

13. The sunscreen composition of claim 1, further including an emulsifying agent.

14. The sunscreen composition of claim 1, wherein the sunscreen composition is in the form of a lotion, a spray, or combinations thereof.

15. A sunscreen composition comprising:

    an anhydrous combination comprising:

    a mineral sunscreen agent, wherein the mineral sunscreen agent includes titanium dioxide present in an amount up to and including 20 wt. %, based on a total weight of the sunscreen composition, zinc oxide present in an amount of 5 wt. % to 30 wt. %, based on a total weight of the sunscreen composition, or combinations thereof;
    a film former selected from polyester copolymers, wherein the polyester copolymers include octyldodecyl citrate crosspolymer, and wherein the polyester copolymers are present in an amount of 0.05 wt. % to 5 wt. %, based on a total weight of the sunscreen composition; and

a non-aqueous diluent, wherein the non-aqueous diluent is present in an amount up to and including 95 wt. %, based on a total weight of the sunscreen composition; and
an emulsifying agent, wherein the sunscreen composition is in the form of a lotion, a spray, or combinations thereof.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62044490